# EUROPEAN PATENT APPLICATION

(11) **EP 4 306 120 A1**
(43) Date of publication of application: **17.01.2024**
(21) Application number: 21930170.2
(22) Date of filing: 11.03.2021
(51) Int. Cl.: A61K 35/742, A61P 31/12, A61P 31/16, A61P 43/00

(54) **INTERFERON PRODUCTION PROMOTER**

(71) Applicant: Miyarisan Pharmaceutical Co., Ltd., Sakaki-machi Hanishina-gun Nagano 389-0682 (JP)
(72) Inventor: HAGIHARA, Mao, Nagakute-shi, Aichi 480-1195 (JP); MIKAMO, Hiroshige, Nagakute-shi, Aichi 480-1195 (JP); YAMAGISHI, Yuka, Nagakute-shi, Aichi 480-1195 (JP); YAMASHITA, Makoto, Nagakute-shi, Aichi 480-1195 (JP); TAKAHASHI, Motomichi, Tokyo 114-0016 (JP); OKA, Kentaro, Tokyo 114-0016 (JP); ARIYOSHI, Tadashi, Tokyo 114-0016 (JP)
(74) Representative: J A Kemp LLP
(86) International application number: PCT/JP2021/009831
(87) International publication number: WO 2022/190317

(57) **Abstract**

[Problem] Provided is a novel interferon production promoter.

[Solution] A type I and/or type III interferon production promoter contains Clostridium butyricum or a culture thereof as an active ingredient.

## Description

### TECHNICAL FIELD

The present invention relates to an interferon production promoter.

### BACKGROUND ART

Interferon (IFN) is a physiologically active protein produced by various cells such as lymphocytes, fibroblasts, and the like in vivo. Interferon is classified into type I interferons, type II interferons, and type III interferons based on recognition of protein structures and receptor complexes. Interferon exhibits various physiological activities such as an antiviral action, an anticancer action, and the like, and among them, it is known that the antiviral action is an action by type I and type III interferons.

So far, compositions containing bacteria or cultures thereof have been studied to promote production of type I and type III interferons. For example, WO 2012/091081 A discloses an IFN production inducer containing lactic acid bacteria or a culture thereof as an active ingredient.

### SUMMARY OF INVENTION

An object of the present invention is to provide a novel type I and/or type III interferon production promoter.

The present inventors have surprisingly found that Clostridium butyricum promotes production of interferons. Based on this finding, the present inventors have completed the present invention.

That is, according to an aspect of the present invention, a type I and/or type III interferon production promoter containing Clostridium butyricum or a culture thereof as an active ingredient is provided.

### BRIEF DESCRIPTION OF DRAWINGS

Fig. 1 illustrates a schematic view of a test in Examples.
Fig. 2 is a graph showing the results of changes in survival time and body weight in a CBM588 administration group and a control group.
Fig. 3 illustrates the measurement results of the amount of influenza virus in lung tissues in the CBM588 administration group and the control group.
Fig. 4 illustrates the measurement results of concentrations of interferon α, interferon β, and interferon λ in bronchoalveolar lavage fluid in the CBM588 administration group and the control group.
Fig. 5 illustrates the measurement results of concentrations of secretory IgA in bronchoalveolar lavage fluid and blood in the CBM588 administration group and the control group.

### DESCRIPTION OF EMBODIMENTS

Hereinafter, embodiments according to an aspect of the present invention will be described. The present invention is not limited to only the following embodiments.

In the present specification, "X to Y" representing a range means "X or more and Y or less". In addition, unless otherwise specified, an operation and a measurement of physical properties and the like are carried out under a condition of room temperature (20 to 25°C)/relative humidity of 40 to 50%RH.

### <Interferon Production Promoter>

An aspect of the present invention is a type I and/or type III interferon production promoter containing Clostridium butyricum or a culture thereof as an active ingredient.

In the present specification, the "type I and/or type III interferon production promoter" is also simply referred to as an "interferon production promoter".

Interferon (IFN) is a physiologically active protein produced by various cells such as lymphocytes, fibroblasts, and the like. Interferon is classified into type I interferons, type II interferons, and type III interferons based on recognition of protein structures and receptor complexes. The type I interferon includes IFN-α, IFN-β, IFN-ω, IFN-ε, IFN-κ, and the like. The type II interferon is composed of only of IFN-γ. The type III interferon includes IFN-λ.

The promotion of production of the type I and/or type III interferon means that production of at least one of type I and type III interferons in vivo (for example, in bronchoalveolar lavage fluid) can be promoted.

In one embodiment, the interferon production promoter according to the present invention can promote the production of at least one of a type I interferon and a type III interferon, and in particular, can promote the production of at least one selected from the group consisting of IFN-α, IFN-β, and IFN-λ.

Clostridium butyricum is a spore-forming anaerobic gram-positive bacillus that repeatedly divides and proliferates (vegetative cells) while a nutritional balance is maintained, and produces spores within the bacteria when the balance is disrupted. Not only anaerobic bacteria, but many bacteria having morphology of vegetative cells easily die when left in a dry state. However, since spores are quiescent cells, the spores have strong resistance to various external environments such as drying, heat, chemicals, and the like, and are advantageous for storage.

In addition, as described above, Clostridium butyricum is spore-forming and has resistance to various external environments when Clostridium butyricum is in a spore state. Therefore, when Clostridium butyricum is orally administered to a human or an animal in the form of spores, even when it comes into contact with digestive fluids such as gastric acid, intestinal fluid, bile acid, and the like, Clostridium butyricum is not completely killed, and it is possible to reach a fermentation site from the lower part of the small intestine to the large intestine and proliferate.

Furthermore, Clostridium butyricum is widely marketed as a probiotic agent, a feed additive, or a food, and has no side effect at all even when administered to mammals such as humans, livestock, and the like for a long period of time, and is guaranteed to be highly safe.

Among Clostridium butyricum, Clostridium butyricum MIYAIRI, Clostridium butyricum NIP1020, Clostridium butyricum NIP1021, Clostridium butyricum (FERM P-11868), Clostridium butyricum (FERM P-11868), Clostridium butyricum (FERM P-11869), and Clostridium butyricum (FERM P-11870), Clostridium butyricum ATCC859, Clostridium butyricum NBRC3315, Clostridium butyricum ATCC860, or Clostridium butyricum ATCC19398 is preferable. The Clostridium butyricum is more preferably one or more selected from the group consisting of Clostridium butyricum MIYAIRI 588 (FERM BP-2789), Clostridium butyricum MIYAIRI 585 (FERM BP-06815), Clostridium butyricum MIYAIRI 595 (FERM BP-06816), and Clostridium butyricum MIYAIRI 630 (FERM BP-06817), and still more preferably Clostridium butyricum MIYAIRI 588 (FERM BP-2789) . Note that Clostridium butyricum MIYAIRI 588 strain was deposited as FERM BP-2789 with Fermentation Research Institute, Agency of Industrial Science and Technology (currently called National Institute of Technology and Evaluation, International Patent Organism Depositary) (2-5-8 Kazusakamatari, Kisarazu, Chiba 292-0818, Japan) on May 1, 1981, and was transferred to the International Depositary Authority under the Budapest Treaty on March 6, 1990 and was deposited as accession number FERM BP-2789.

Clostridium butyricum MIYAIRI is commercially available as a probiotic agent from Miyarisan Pharmaceutical Co., Ltd., and is particularly suitable for use in the present invention since it has no side effects even when administered to humans or animals for a long period of time. Note that, as Clostridium butyricum, which is an active ingredient, only one kind may be used alone, or two or more kinds may be used in combination.

In the present invention, the culture of Clostridium butyricum means a culture solution in which Clostridium butyricum is cultured, a residue containing bacteria obtained by centrifuging the culture solution, or a dried product of the residue.

The culture of Clostridium butyricum is obtained by a known method for culturing a microorganism, for example, a method disclosed in JP H08-252088 A. One embodiment is shown below: a "culture solution of Clostridium butyricum" is obtained by inoculating 10⁵ to 10⁶ cells/mL of Clostridium butyricum in a medium containing 1.0 (w/v)% of a peptone, 1.0 (w/v)% of a yeast extract, 1.0 (w/v)% of cornstarch, and 0.2 (w/v)% of precipitated calcium carbonate, and statically culturing the inoculated cells at 37°C for 48 hours. Next, the resulting culture solution is centrifuged (2,000 to 6,000 g for 10 to 30 minutes) to separate a "residue containing bacteria obtained by centrifuging the culture solution", and the residue is subjected to a drying treatment by air drying or the like at 0 to 80°C and preferably 10 to 20°C for 1 to 24 hours and preferably 5 to 18 hours, or a drying treatment under reduced pressure at 0 to 80°C and preferably 10 to 20°C, and 0.05 to 500 Torr (7 Pa to 66.7 kPa) and preferably 1 to 100 Torr (133 Pa to 13.3 kPa) for 1 to 24 hours and preferably 2 to 15 hours, thereby obtaining a "dried product of the residue". In order to obtain a dried product, spray drying, freeze drying, or the like may be used.

The medium used for culturing Clostridium butyricum according to the present invention varies depending on the type of strain to be used and the like, and may be either a synthetic medium or a natural medium as long as it is a medium containing a carbon source that can be assimilated by Clostridium butyricum to be used, an appropriate amount of a nitrogen source, an inorganic salt, and other nutrients such as vitamins.

For example, the carbon source used in the medium according to the present invention is not particularly limited as long as a strain to be used is a carbon source that can be assimilated. The carbon source is not necessarily limited to a sugar, and in consideration of proliferation of bacteria, a sugar or a carbon source containing a sugar available to bacteria to be used is preferably used. Specific examples of the carbon source that can be used include cellobiose, glucose, fructose, galactose, lactose, maltose, mannose, melibiose, raffinose, salicin, starch, sucrose, trehalose, xylose, dextrin, molasses, and the like, in consideration of assimilability. Among these carbon sources, starch, glucose, fructose, sucrose, and molasses are preferably used. One or two or more of the carbon sources described above may be selected and used in consideration of Clostridium butyricum to be used. At this time, a concentration of the carbon source to be added is usually 0.5 to 5 (w/v)% and preferably 2 to 4 (w/v)%, although the concentration varies depending on the type of Clostridium butyricum or the carbon source to be used, a medium composition of the medium to be used other than the carbon source, and the like.

In addition, examples of the nitrogen source and the vitamins include a meat extract, a peptone, a yeast extract, soybean and wheat hydrolysates such as a flavor liquid, soybean powder, milk casein, a casamino acid, various amino acids, corn steep liquor, organic nitrogen compounds such as other animal, plant, and microbial hydrolysates, ammonium salts such as ammonium sulfate and the like. Among these nitrogen sources, a peptone, a yeast extract, a meat extract, corn steep liquor, and a flavor liquid are preferably used. One or two or more of the nitrogen sources and the vitamins described above may be selected and used in order to improve the growth of Clostridium butyricum to be used. At this time, a concentration of the nitrogen source added varies depending on the type of the strain or the nitrogen source to be used, a medium composition of the medium to be used other than the nitrogen source, and the like, and is usually 0.5 to 4 (w/v)% and preferably 1 to 3 (w/v) % when a peptone containing a large amount of a nitrogen source is used, is usually 0.5 to 5 (w/v) % and preferably 1 to 4 (w/v) % when a flavor liquid or a corn steep liquor containing a large amount of a nitrogen source and vitamins is used, and is usually 0.5 to 4 (w/v)% and preferably 1 to 3 (w/v)% when a yeast extract or a meat extract containing a large amount of vitamins is used.

In addition, as the inorganic salt, one or two or more of inorganic salts selected from phosphates, hydrochlorides, sulfates, butyrates, propionates, acetates, and the like of magnesium, manganese, calcium, sodium, potassium, molybdenum, strontium, boron, copper, iron, tin, zinc, and the like can be used. In addition, an antifoaming agent, a vegetable oil, a surfactant, blood, and a blood component, a drug such as antibiotics, a physiologically active substance such as a plant or animal hormone, and the like may be appropriately added to the medium, if necessary.

The conditions of the culture performed in the present invention vary depending on physiological properties such as the range of growth (pH, temperature, or the like) of Clostridium butyricum used in the present invention, but since Clostridium butyricum is strictly anaerobic, Clostridium butyricum should be cultured under anaerobic conditions without aeration or with aeration of nitrogen or carbon dioxide gas, or by lowering an oxidation-reduction potential by adding a reducing agent to the medium, or the like. The culture conditions at this time are appropriately selected depending on the range of growth of the strain to be used, the composition of the medium, and the culture method, and are not particularly limited as long as the conditions are that the present strain can proliferate. Specifically, the culture temperature is usually 20 to 42°C and preferably 35 to 40°C.

In addition, according to the present invention, in the culture of Clostridium butyricum, since the proliferation is promoted by neutralizing the acid produced during the culture with an alkali, it is preferable to add calcium carbonate to the medium in advance. At this time, the amount of calcium carbonate added is usually 0.1 to 4 (w/v)% and preferably 0.2 to 2.5 (w/v)%. Alternatively, it is also preferable that the neutralization step is performed while the pH of the medium is kept within the set pH range by an alkali aqueous solution such as sodium hydroxide, sodium hydrogen carbonate, sodium carbonate, potassium hydroxide, potassium carbonate, or the like. Note that, in a case where an alkali aqueous solution is used, the "set pH" means the pH of the medium set in advance during the culture period, and the "range of the set pH" is the pH range allowed during the culture period and is generally represented by the set pH ± allowable difference. According to the present invention, the set pH is usually set within a range of 5.0 to 7.5 and preferably 5.5 to 6.5, and the range of the set pH is the set pH ± 0.5 and preferably the set pH ± 0.2.

Note that, in the present invention, the pH of the medium during culture is set to near neutral at the time of inoculation of bacteria, and is more preferably 6.5 to 7.5. Note that, in a case where an alkali aqueous solution is used, it is preferable to maintain the pH within the set pH range while gently stirring so that oxygen is not mixed. By controlling the pH at the time of inoculation of bacteria and at the time of proliferation of bacteria as described above, a bacterial density can be dramatically increased.

In the culture according to the present invention, an initial culture concentration of Clostridium butyricum is not particularly limited as long as it is within a range in which Clostridium butyricum can grow, and is usually the same as that performed in the culture of Clostridium butyricum. Specifically, the initial culture concentration of Clostridium butyricum is usually 10⁴ to 10⁷ cells/mL and preferably 10⁵ to 10⁶ cells/mL.

The culture of Clostridium butyricum thus obtained, in particular, the culture of Clostridium butyricum MIYAIRI 588 (FERM BP-2789), has a high ability to promote production of interferons.

As shown in Examples described below, the production of a type I interferon and a type III interferon, in particular, IFN-α, IFN-β, and IFN-λ is promoted by Clostridium butyricum or a culture thereof according to the present invention. It is considered that an antiviral action in vivo can be enhanced by promoting production of interferon. The interferon production promoting action is an action that has not been known as an effect of Clostridium butyricum.

Another aspect of the present invention is Clostridium butyricum or a culture thereof for use as an interferon production promoter.

Still another aspect of the present invention is Clostridium butyricum or a culture thereof for production of an interferon production promoter.

The interferon production promoter according to the present invention contains Clostridium butyricum or a culture thereof in an amount of sufficient to exhibit a desired effect (that is, an effective amount). The interferon production promoter may be Clostridium butyricum or a culture thereof itself, or may be prepared as an oral preparation or a parenteral preparation in combination with an additive acceptable for formulation according to a normal method. The interferon production promoter is preferably an oral preparation from the viewpoint of simplicity. Examples of the additive acceptable for formulation can include an excipient, a stabilizer, an antiseptic agent, a wetting agent, an emulsifier, a lubricant, a sweetener, a colorant, a flavor, a buffer, an antioxidant, a pH adjusting agent, a binder, a thickener, a dispersant, a suspending agent, a disintegrant, a bacteriostatic agent, a surfactant, and the like. A dosage form is not particularly limited and may be appropriately set, and examples thereof include tablets, powders, fine granules, granules, capsules, pills, sustained release agents, solutions, suspensions, emulsions, lotions, injections, drops, external preparations, suppositories, patches, and the like.

The interferon production promoter according to the present invention can be administered to mammals or birds, and preferably mammals or birds suffering from or having a possibility of a virus infection described below. Here, the mammals include both primates such as a human, a monkey, a gorilla, a chimpanzee, an orangutan, and the like, and non-human mammals such as a mouse, a rat, a hamster, a guinea pig, a rabbit, a dog, a cat, a pig, a cow, a horse, a sheep, a camel, a goat, and the like. Examples of the birds include a chicken, a quail, a pigeon, and the like. Among them, a human is preferable.

### <Method for Promoting Production of Interferon>

An aspect of the present invention is a method for promoting production of interferon, the method including administering an effective amount of Clostridium butyricum or a culture thereof to a subject.

Since the "Clostridium butyricum or a culture thereof" is the same as described in the interferon production promoter, the description thereof is omitted.

In the present aspect, the "effective amount" means an amount of an active ingredient (that is, Clostridium butyricum or a culture thereof) at least required for exhibiting an effect of promoting production of interferon.

In the present aspect, the "subject" is not particularly limited, and is mammals or birds and preferably mammals or birds suffering from or having a possibility of a virus infection described below. The mammals and birds are the same as the matters described in the interferon production promoter according to the present invention, and thus the description thereof is omitted. In the present aspect, the subject is preferably a human.

In the present aspect, Clostridium butyricum or a culture thereof may be administered by oral, intravenous, intramuscular, intrathecal, intraperitoneal, percutaneous (for example, as an ointment), or inhalation administration. Clostridium butyricum or a culture thereof may be prepared as an oral preparation or a parenteral preparation according to each of these administration forms in combination with an additive acceptable for formulation according to a normal method. The additive acceptable for formulation is the same as the matters described in the interferon production promoter according to the present invention, and thus the description thereof is omitted.

### <Agent for Preventing and/or Treating Virus Infection>

An aspect of the present invention is an agent for preventing and/or treating a virus infection containing the interferon production promoter described above as an active ingredient.

As described above, the interferon production promoter containing Clostridium butyricum or a culture thereof as an active ingredient promotes production of a type I interferon and a type III interferon, in particular, IFN-α, IFN-β, and IFN-λ. Furthermore, as shown in Examples described below, the interferon production promoter according to the present invention increases a concentration of IgA in lung tissues and blood (see Examples). Therefore, the interferon production promoter according to the present invention can exhibit an antiviral action (for example, an anti-influenza virus action) by containing Clostridium butyricum or a culture thereof as an active ingredient.

Examples of the virus infection include infections such as influenza virus, a coronavirus including SARS-CoV-2, hepatitis C virus (HCV), herpes virus, papilloma virus, RS virus, norovirus, rotavirus, and the like. The interferon production promoter according to the present invention can exhibit an effect for preventing and/or treating particularly an influenza virus infection.

The form of the agent for preventing and/or treating a virus infection may be administered by any route of administration deemed appropriate by those skilled in the art. For example, the agent for preventing and/or treating a virus infection may be administered by oral, intravenous, intramuscular, intrathecal, intraperitoneal, percutaneous (for example, as an ointment), or inhalation administration. The agent for preventing and/or treating a virus infection contains a pharmacologically acceptable carrier according to each of these administration forms. The pharmacologically acceptable carrier is not particularly limited, and examples thereof include an excipient such as lactose and starch; a binder such as dextrin and cellulose; a solvent such as water and an organic solvent; and a base such as petrolatum, beeswax and paraffin, or the like.

A blending ratio of the active ingredient in the agent for preventing and/or treating a virus infection is not particularly limited. The agent for preventing and/or treating a virus infection preferably contains an appropriate amount of an interferon production promoter so as to contain an active ingredient of an interferon production promoter, that is, an effective amount of Clostridium butyricum or a culture thereof. The blending ratio of the interferon production promoter is, for example, 0.001 to 50 mass% of the interferon production promoter with respect to the entire agent for preventing and/or treating a virus infection.

Note that the present aspect includes the following embodiments:
an agent for preventing and/or treating a virus infection containing Clostridium butyricum or a culture thereof as an active ingredient;
use of Clostridium butyricum or a culture thereof for use as an agent for preventing and/or treating a virus infection; and
use of Clostridium butyricum or a culture thereof for preparation of an agent for preventing and/or treating a virus infection.

The agent for preventing and/or treating a virus infection according to the present invention can contain other auxiliary components, if necessary. Examples of the other auxiliary components include an antibiotic, vitamins (for example, vitamin C and vitamin E), amino acids, peptides, minerals (for example, zinc, iron, copper, manganese, and the like), a nucleic acid, polysaccharides, fatty acids, a crude drug, and the like.

A dosage and administration of the agent for preventing and/or treating a virus infection according to the present invention may be appropriately changed depending on symptom and disease state to be treated, age, and the like, and is, for example, 0.1 to 1,000 mg/kg body weight as an active ingredient.

A subject of the agent for preventing and/or treating a virus infection according to the present invention is mammals or birds, and preferably mammals or birds suffering from or having a possibility of the virus infection described above. The mammals and birds are the same as the matters described in the interferon production promoter according to the present invention, and thus the description thereof is omitted. In the present aspect, the subject is preferably a human.

### <Method for Preventing and/or Treating Virus Infection>

An aspect of the present invention is a method for preventing and/or treating a virus infection, the method including administering an effective amount of the interferon production promoter to a subject in need thereof.

In the present aspect, the "subject" and the "virus infection" are the same as the matters described in the agent for preventing and/or treating a virus infection, and thus the description thereof is omitted.

In the present aspect, the "effective amount" means an amount of an active ingredient of the interferon production promoter (that is, Clostridium butyricum or a culture thereof) at least required for exhibiting a desired effect of preventing and/or treating a virus infection.

Note that the present aspect includes the following embodiments.

A method for preventing and/or treating a virus infection includes administering an effective amount of Clostridium butyricum or a culture thereof to a subject in need thereof.

In the present aspect, a dosage of the interferon production promoter can be appropriately changed depending on symptom and disease state to be treated, age, and the like.

### <Food and Beverage Product>

An aspect of the present invention is a food and beverage product containing the interferon production promoter described above.

The food and beverage product according to the present invention preferably contains an appropriate amount of an interferon production promoter so as to contain an active ingredient of an interferon production promoter, that is, an effective amount of Clostridium butyricum or a culture thereof. In the present aspect, the "effective amount" means that an active ingredient is contained in an amount in which an effect as an active ingredient is exhibited as a result of intake of an amount of an individual food and beverage product that is usually eaten.

The food and beverage product according to the present invention may be a food and beverage product produced by adding a conventional additional component such as a stabilizer to the interferon production promoter, a food and beverage product produced by further blending various proteins, saccharides, fats, trace elements, vitamins, and the like thereinto, a liquid, semi-liquid, or solid food and beverage product, a pasty food and beverage product, or a food and beverage product obtained by adding an interferon production promoter to a general food and beverage product.

In the present invention, the "food and beverage product" is not particularly limited as long as it is a product other than a medicine and is in a form that can be orally ingested by a mammal or the like, and the form may be any of a liquid product (a solution, a suspension, an emulsion, or the like), a semi-liquid product, a powder, and a solid molded product. Therefore, the food and beverage product may be in the form of, for example, a beverage, or may be in the form of a tablet of a nutritional supplement such as a supplement.

Specific examples of the food and beverage product include instant foods such as instant noodles, retort foods, canned foods, microwave oven foods, instant soups/miso soups, freeze-dried foods, and the like; beverages such as soft beverages, fruit juice beverages, vegetable beverages, soy milk beverages, coffee beverages, tea beverages, powdered beverages, concentrated beverages, nutritional beverages, alcoholic beverages, and the like; flour products such as bread, pasta, noodles, cake mixes, fried chicken flour, bread crumbs, and the like; confectionery such as candies, caramels, chewing gums, chocolates, cookies, biscuits, cakes, pies, snacks, crackers, Japanese sweets, desserts, and the like; seasonings such as sauces, tomato processed seasonings, flavor seasonings, cooking mixes, spicery, dressings, soups, curry and stew ingredients, and the like; fats and oils such as processed fats, butter, margarine, mayonnaise, and the like; dairy products such as milk beverages, yogurts, lactic acid beverages, ice creams, creams, and the like; processed marine products such as fish meat hams and sausages, fish paste products, and the like; processed livestock products such as meat hams and sausages and the like; processed agricultural products such as canned agricultural products, jams and marmalades, pickles, cooked beans, cereals, and the like; frozen food; and nutritional food and the like.

In the present invention, the "food and beverage product" also includes foods classified as health foods, functional foods, foods for specified health uses, nutritional supplementary foods, foods labeled with disease risk reduction, or foods for patients. Furthermore, the term "food and beverage product" when used for mammals other than humans and birds may be used in the sense of including feed.

In the food and beverage product of the present invention, a component having another function may be further added in addition to the active ingredient described above. In addition, for example, the active ingredient of the present invention is blended with foods, health foods, functional foods, and supplements (for example, foods containing one or more minerals such as calcium, magnesium, and the like, and vitamins such as vitamin K and the like) taken in daily life, such that it is possible to provide a food and beverage product having a function based on other ingredients in addition to the effect according to the present invention.

A blending ratio of the interferon production promoter in the food and beverage product is not particularly limited, and the interferon production promoter is, for example, 0.001 to 50 mass% with respect to a mass of the dried food and beverage product.

### EXAMPLES

Hereinafter, the present invention will be described by using specific Examples, but the present invention is not limited by these Examples. Note that, unless otherwise noted, the operation was performed at room temperature (25°C).

Animal experiments using mice were performed with the approval of the Institutional Animal Care and Use Committee, ethics committee established by Aichi Medical University (approval number: 2020-38).

### <Preparation of Influenza Virus-Infected Model Mouse>

Balb/c mice (9 to 10 weeks-old, female, purchased from Charles River Laboratories Japan, Inc.) were provided for a 7-day quarantine and acclimation period, and the Balb/c mice were subjected to a test after confirming that there was no abnormality in the health condition of all individuals and no body weight loss was observed. Mice were allowed to freely take food and water in a breeding environment of lighting for 12 hours, a temperature of 20 to 26°C, and a humidity of 30 to 70%.

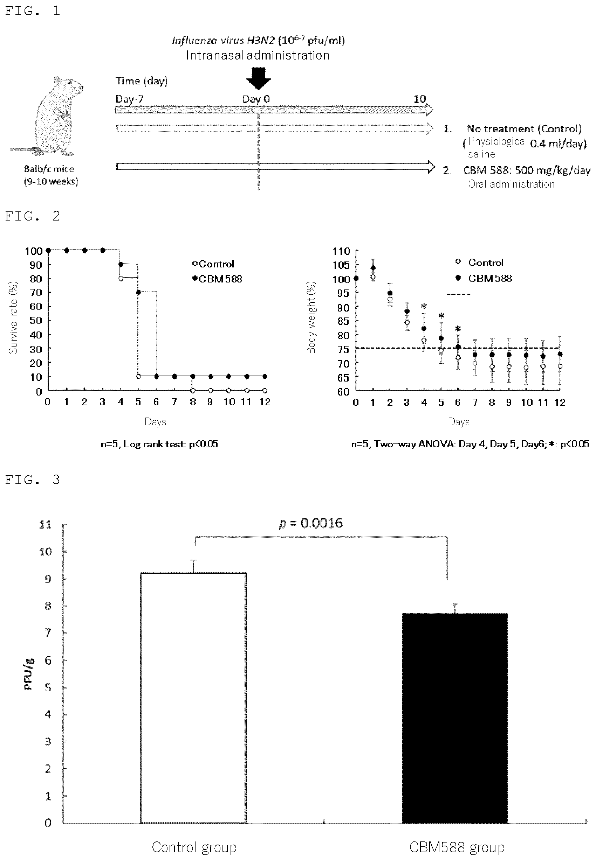

Mice were intranasally infected with influenza virus H3N2 (1.8 × 10⁵ pfu/mL) to prepare an influenza virus-infected model mouse 1. In addition, mice were intranasally infected with influenza virus H3N2 (1.8 × 10⁴ pfu/mL) to prepare an influenza virus-infected model mouse 2 (see Fig. 1).

### <Test Example 1: Evaluation of Changes in Survival Time and Body Weight>

In a CBM588 administration group, a culture of Clostridium butyricum MIYAIRI 588 (FERM BP-2789) suspended in physiological saline (hereinafter, also simply referred to as "CBM588") was orally administered to the influenza virus-infected model mouse 1 at 500 mg/kg/day (n = 5). The amount of bacteria in CBM588 was 3.3 × 10¹⁰ cfu/g. Note that CBM588 is a dried product of a residue obtained by subjecting Clostridium butyricum MIYAIRI 588 to static culture at 37°C for 48 hours using a CS liquid medium containing 2.0 (w/v)% of corn starch, 2.0 (w/v)% of an amino acid solution, and 0.75 (w/v)% of calcium carbonate, and then centrifuging the culture solution.

In a control group, the influenza virus-infected model mouse 1 was orally administered with physiological saline at 0.4 mL/day (n = 5).

While the health condition of the mice was observed, the mice were allowed to freely take food and water in a breeding environment of lighting for 12 hours, a temperature of 20 to 26°C, and a humidity of 30 to 70%, and changes in survival time and body weight were compared between the CBM588 administration group and the control group. The results are illustrated in Fig. 2.

As illustrated in Fig. 2, compared with the control group, in the CBM588 administration group, prolongation of survival time and a suppression effect of body weight loss were observed.

### <Test Example 2: Measurement of Amount of Influenza Virus in Lung Tissues>

In the CBM588 administration group, CBM588 suspended in physiological saline was orally administered to the influenza virus-infected model mouse 2 at 500 mg/kg/day (n = 5). In a control group, the influenza virus-infected model mouse 2 was orally administered with physiological saline at 0.4 mL/day (n = 5) .

As in Test Example 1, the mice were bred while the health condition of the mice was observed.

Mice were euthanized on day 2 after administration. Lung tissues were collected and homogenized, and then a plaque assay was performed using the supernatant to measure the amount of virus. The results are illustrated in Fig. 3.

As illustrated in Fig. 3, compared with the control group, in the CBM588 administration group, a significant reduction in the amount of influenza virus in the lung tissues was observed.

### <Test Example 3: Measurement of Interferon Concentration in Bronchoalveolar Lavage Fluid>

In the CBM588 administration group, CBM588 suspended in physiological saline was orally administered to the influenza virus-infected model mouse 2 at 500 mg/kg/day (n = 5). In a control group, the influenza virus-infected model mouse 2 was orally administered with physiological saline at 0.4 mL/day (n = 5) .

As in Test Example 1, the mice were bred while the health condition of the mice was observed.

Mice were euthanized on day 1, 2, 4 or 7 after administration. The trachea of the mouse was incised, and the lung was washed with PBS to obtain a bronchoalveolar lavage fluid (BALF) sample.

A concentration of IFN-α, β, and λ in the BALF sample was measured by ELISA method using the following kits:
IFN-α: VeriKine^{™} IFN-α measurement ELISA kit (mouse), manufactured by PBL, #42120-1,
IFN-β: Mouse IFN-beta Quantikine ELISA Kit, manufactured by RSD, #MIFNB0, and
INF-λ: Mouse IL-28 ELISA Kit, manufactured by abcam, #ab100708.

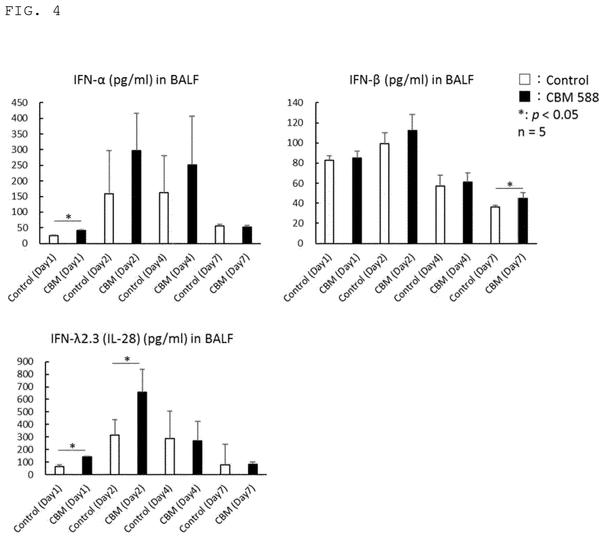

The results are illustrated in Fig. 4.

As illustrated in Fig. 4, it could be seen that the interferon concentration was increased in the CBM588 administration group as compared with the control group.

### <Test Example 4: Measurement of Concentrations of Secretory IgA in BALF and Blood>

In the CBM588 administration group, CBM588 suspended in physiological saline was orally administered to the influenza virus-infected model mouse 2 at 500 mg/kg/day (n = 5). In a control group, the influenza virus-infected model mouse 2 was orally administered with physiological saline at 0.4 mL/day (n = 5) .

As in Test Example 1, the mice were bred while the health condition of the mice was observed.

Mice were euthanized on day 1, 2, 3, 4 or 7 after administration. The trachea of the mouse was incised, and the lung was washed with PBS to obtain a bronchoalveolar lavage fluid (BALF) sample. In addition, a blood sample was collected by cardiac puncture.

Concentrations of secretory IgA in BALF and blood were measured by ELISA method using the following kit:
IgA: IgA Mouse ELISA kit, manufactured by Thermofisher, #EMIGA.

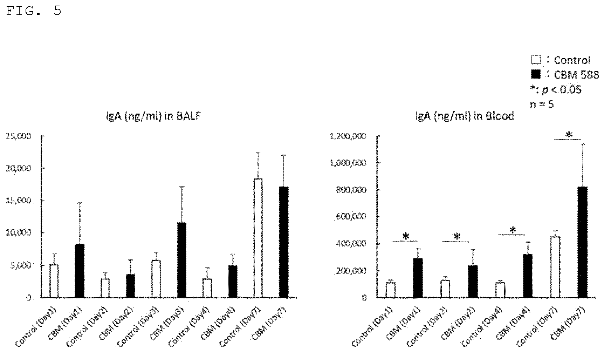

The results are illustrated in Fig. 5.

As illustrated in Fig. 5, it could be seen that the concentration of secretory IgA was increased in the CBM588 administration group as compared with the control group.

## Claims

1. A type I and/or type III interferon production promoter, comprising Clostridium butyricum or a culture thereof as an active ingredient.

2. The interferon production promoter according to claim 1, wherein the Clostridium butyricum is Clostridium butyricum MIYAIRI 588 (FERM BP-2789) .

3. The interferon production promoter according to claim 1 or 2, wherein the interferon is at least one selected from the group consisting of IFN-α, IFN-β, and IFN-λ.

4. An agent for preventing and/or treating a virus infection, comprising the interferon production promoter according to any one of claims 1 to 3 as an active ingredient.

5. The agent for preventing and/or treating a virus infection according to claim 4, wherein the virus infection is an influenza virus infection.

6. A method for preventing and/or treating a virus infection, comprising administering the interferon production promoter according to any one of claims 1 to 3 to a subject in need thereof.

7. A method for promoting production of an interferon, comprising administering an effective amount of Clostridium butyricum or a culture thereof to a subject.

8. Clostridium butyricum or a culture thereof for use as an interferon production promoter.

9. Use of Clostridium butyricum or a culture thereof for production of an interferon production promoter.

10. A food and beverage product comprising the interferon production promoter according to any one of claims 1 to 3.
